# DEMANDE DE BREVET EUROPEEN

(11) **EP 0 688 750 A1**
(43) Date de publication de la demande: **27.12.1995**
(21) Numéro de dépôt: 95401356.1
(22) Date de dépôt: 12.06.1995
(51) Int. Cl.: C07C 2/62

(54) **Procédé d'alkylation d'isoparaffine**

(30) Priorité: 23.06.1994 FR 9407857
(71) Demandeur: INSTITUT FRANCAIS DU PETROLE, F-92502 Rueil-Malmaison (FR)
(72) Inventeur: Benazzi, Eric, F-78360 Montesson (FR); Boucot, Pierre, F-69360 Ternay (FR); Duplan, Jean-Luc, F-69540 Irigny (FR); Joly, Jean-François, F-69006 Lyon (FR)

(57) **Abrégé**

Procédé d'alkylation d'au moins une oléfine par au moins une isoparaffine en présence d'un catalyseur d'alkylation sous forme de particules solides dans lequel la réaction est effectuée dans une zone d'alkylation contenant une pluralité de conteneurs renfermant chacun une quantité de particules solides catalytiques telle que le volume Va de ces particules mesuré après leur mise en contact avec les réactifs dans les conditions de la réaction d'alkylation soit supérieur à zéro et inférieur ou égal au volume interne Vt dudit conteneur, lesdits conteneurs ayant chacun une enveloppe externe perméable aux fluides et imperméable auxdites particules solides catalytiques et ayant des caractéristiques mécaniques suffisantes pour résister, sans déformation excessive, à la charge de l'ensemble des conteneurs présents dans ladite zone d'alkylation.

## Description

La présente invention concerne un procédé d'alkylation d'au moins une oléfine par au moins une isoparaffine en présence d'un catalyseur d'alkylation sous forme de particules solides.

Pour alimenter les moteurs à combustion interne et à allumage commandé, et notamment les moteurs à taux de compression élevé, il est particulièrement intéressant de disposer de carburants à haut indice d'octane, c'est à dire comprenant essentiellement des hydrocarbures paraffiniques fortement ramifiés. L'alkylation d'isoparaffines (par exemple l'isobutane et/ou l'isopentane) par les oléfines contenant de préférence de 3 à 6 atomes de carbone par molécule permet d'obtenir de tels produits. Cette réaction nécessite la mise en oeuvre de catalyseurs très acides, dans le but notamment de réduire les réactions parasites telles que les réactions de dimérisation et de polymérisation de l'oléfine, qui fournissent des hydrocarbures moins ramifiés et des hydrocarbures insaturés, les réactions de craquage et les réactions de dismutation.

Les procédés industriels existants pour la production d'hydrocarbures par alkylation d'isoparaffines (en particulier d'isobutane) par les oléfines utilisent comme catalyseur soit l'acide sulfurique, soit l'acide fluorhydrique. Dans ces procédés, le catalyseur acide constitue une phase liquide qui est mise en contact avec le mélange isoparaffine(s)-oléfine(s) liquide pour former une émulsion. Ces procédés posent d'importants problèmes de rejets polluants ainsi que de sécurité.

D'autres procédés existent également qui utilisent des catalyseurs acides hétérogènes tels que des tamis moléculaires, des résines macroréticulaires éventuellement associées avec le trifluorure de bore (BF₃), des résines perfluorées, des acides de Lewis et/ou de Brönsted et des anions déposés sur des supports oxydes tels que par exemple ZrO₂/SO₄.

Ces catalyseurs peuvent être mis en oeuvre de différentes manières :
- a/ dans un réacteur de type Grignard. Dans ce cas, le catalyseur est mis en suspension dans la phase hydrocarbonée par agitation vigoureuse au sein du réacteur. On peut citer à titre d'exemple les procédés de ce type décrit dans les brevets US-A- 3 879 489 et US-A- 3 855 343. Ce type de mise en oeuvre présente un inconvénient majeur : le catalyseur peut être rapidement détruit par attrition.
- b/ dans un réacteur à slurry entraîné. Cette technique est par exemple décrite dans les brevets US-A-5 190 904 et US-A-5 157 196. Elle nécessite des dispositifs de séparation du catalyseur des produits de réaction. On peut de ce point de vue envisager des dispositifs de type hydrocyclone qui demandent des pertes de charge importantes en contre partie de leur efficacité. Une zone de décantation des particules de catalyseur peut aussi être envisagée à condition que leurs tailles soient suffisamment grandes pour ne pas arriver à des surfaces de décantation irréalistes.
- c/ dans un réacteur en lit fixe. Cette technique est par exemple décrite dans les brevets US-A-3 852 371 et US-A-5 073 665. Le procédé d'alkylation nécessitant de forts débits de liquide (associés aux isoparaffines), Les pertes de charge à travers le lit peuvent rapidement devenir prohibitives pour des particules catalytiques de faibles granulométries.

La présente invention concerne un procédé d'alkylation comprenant une mise en oeuvre particulière d'un catalyseur solide permettant de limiter la perte de charge à l'utilisation, tout en permettant une performance correcte du système catalytique d'un point de vue productivité et sélectivité en produits recherchés.

La présente invention concerne ainsi un procédé d'alkylation d'au moins une oléfine par au moins une isoparaffine en présence d'un catalyseur d'alkylation sous forme de particules solides selon lequel la réaction est effectuée dans une zone d'alkylation contenant une pluralité de conteneurs renfermant chacun une quantité de particules solides catalytiques telle que le volume Va de ces particules mesuré après leur mise en contact avec les réactifs dans les conditions de la réaction d'alkylation soit supérieur à zéro et inférieur ou égal au volume interne Vt dudit conteneur, lesdits conteneurs ayant chacun une enveloppe externe perméable et résistante aux fluides dans les conditions de la réaction et imperméable auxdites particules solides catalytiques et ayant des caractéristiques mécaniques suffisantes pour résister, sans déformation excessive, à la charge de l'ensemble des conteneurs présents dans ladite zone d'alkylation. Ce procédé permet de produire à titre de produit un alkylat contenant une forte proportion d'hydrocarbures paraffiniques fortement ramifiés. Dans le cadre de la présente description les temes "enveloppe résistante aux fluides dans les conditions de la réaction" signifient que dans lesdites conditions le matériau n'est pas attaqué ni dissous par les divers fluides présents dans la zone de réaction ou qu'il n'est attaqué ou dissous que trés lentement et qu'ainsi la durée de vie de l'enveloppe est au moins aussi longue et de préférence plus longue que la durée de vie du catalyseur. Il s'agit en d'autre termes d'une résistance chimique.

Dans le cadre de la présente description, l'expression "sans déformation excessive" signifie que les caractéristiques mécaniques du conteneur sont telles que quelle que soit sa position dans la zone d'alkylation, il continue à pouvoir assurer l'ensemble des fonctions qui lui sont propres et en particulier dans la forme de réalisation préférée décrite ci-dessous à assurer l'existence d'un volume (non nul) vide de tout solide.

Dans une forme préférée de mise en oeuvre de la présente invention la zone d'alkylation contient une pluralité de conteneurs renfermant chacun une quantité de particules solides catalytiques telle que le volume Va de ces particules mesuré après leur mise en contact avec les réactifs dans les conditions de la réaction d'alkylation soit supérieur à zéro et strictement inférieur au volume interne Vt dudit conteneur les renfermant.

Habituellement le catalyseur est choisi dans le groupe formé par : un catalyseur comprenant un support solide organique ou minéral imprégné par au moins un acide minéral et par un catalyseur comprenant au moins un halogénure choisi dans le groupe formé par les halogénures de bore et les halogénures d'aluminium ledit halogénure étant associé à au moins un composé choisi dans le groupe formé par les halogénures d'ammonium quaternaire et les halohydrates d'amine.

Le catalyseur est généralement choisi parmi les catalyseurs solides connus de l'homme de métier et largement décrit dans l'art antérieur concernant cette réaction d'alkylation aliphatique. De préférence, le catalyseur est choisi parmi les catalyseurs suivants :
- un catalyseur comprenant au moins de l'acide sulfurique imprégné sur un support, généralement poreux, organique ou minéral et le plus souvent minéral. A titre d'exemple de tels catalyseurs on peut citer ceux décrits dans les demandes de brevets français au nom de la demanderesse FR-A-2 682 891, FR-A-2 683 740, FR-A-2 683 739 et FR-A-2 687 935.
- un catalyseur comprenant le mélange contenant d'une part au moins un halogénure de composé choisi dans le groupe formé par l'aluminium et le bore et d'autre part au moins un halogénure d'ammonium quaternaire et/ou un halohydrate d'amine, tel par exemple que le catalyseur décrit dans la demande de brevet français au nom de la demanderesse FR-A-2 686 526.

Habituellement le catalyseur est principalement sous forme de particules solides ou grains qui sont le plus souvent sensiblement sphériques et ont habituellement un diamètre moyen d'environ 20x10⁻⁶ à environ 400x10⁻⁶ mètre (m). De préférence le diamètre des particules solides de catalyseur est d'environ 20x10⁻⁶ à environ 150x10⁻⁶ m.

Selon une forme particulière de réalisation de l'invention chaque conteneur comporte quatre faces sensiblement triangulaires constituant sensiblement un tétraèdre et lesdits conteneurs sont disposés en vrac dans la zone d'alkylation. On ne sortirait pas du cadre de la présente invention en disposant les conteneurs de manière ordonnée dans la zone d'alkylation. Le nombre de conteneurs de la zone d'alkylation dépend du volume catalytique nécessaire pour effectuer l'alkylation et du volume réactionnel nécessaire.

Selon une autre forme de mise en oeuvre de l'invention chaque conteneur est relié à au moins un conteneur contigu, de manière à former une chaîne de conteneur et en ce que la zone d'alkylation comprend au moins une chaîne de conteneurs.

Une chaîne de conteneurs comprend une pluralité de conteneurs individuels reliés les uns aux autres, ayant chacun une enveloppe fermée, perméable aux gaz et/ou aux liquides et dont les pores sont suffisamment petits pour retenir les particules solides de catalyseur, chaque conteneur comportant de préférence quatre faces sensiblement triangulaires constituant sensiblement un tétraèdre et chaque conteneur étant relié à au moins un conteneur contigu par l'une de ces arêtes par une liaison commune, de préférence par une soudure commune de deux arêtes adjacentes desdits conteneurs contigus.

Au sens de la présente description le terme pluralité doit être considéré comme équivalent à l'expression au moins deux.

Selon la présente invention, l'enveloppe externe fermée formant le conteneur est perméable aux fluides c'est-à-dire aux liquides et aux gaz et imperméable aux particules solides catalytiques (catalyseur) renfermé(s) dans ledit conteneur. Cette enveloppe empêche ainsi la sortie des particules solides dudit conteneur tout en autorisant le passage des liquides et des gaz à travers ledit conteneur.

L'enveloppe est habituellement réalisée à partir d'un matériau solide qui peut être un matériau perméable ou poreux ou même d'un matériau imperméable dans lequel on a prévu des ouvertures (ou pores) ayant une taille suffisamment petite pour maintenir les particules solides catalytiques à l'intérieur du conteneur et permettant le passage des fluides à travers ledit conteneur.

Il est possible de mieux préciser, les caractéristiques géométriques de ces ouvertures, sans que cela ne soit considéré comme une limitation de la présente invention, en tenant compte de la plus petite dimension de la plus petite des particules solides catalytiques. Dans ce cas, si la plus petite dimension de la plus petite particule solide catalytique est égale à "n" mètre (m), alors la plus grande dimension des ouvertures permettant le passage des fluides sera le plus souvent inférieure ou égale à "0,9xn" m et de préférence inférieure ou égale à "0,5xn" m. Les dimensions des ouvertures permettant le passage des fluides auront de préférence une taille inférieure au diamètre des fines du catalyseur. Dans ces conditions, le catalyseur ne peut s'échapper des conteneurs.

Il n'y a en principe pas de limite pour les dimensions des particules solides catalytiques ou grains de catalyseur et la limite inférieure de la plus grande dimension des ouvertures sera égale à la dimension minimum permettant le passage des fluides et en particulier du liquide. Dans la grande majorité des cas, la zone d'alkylation contiendra des catalyseurs dont la granulométrie peut être comprise entre environ 5x10⁻⁶m et 2x10⁻¹m et le plus souvent dans la gamme granulométrique donnée ci-devant.

Comme exemple de matériau utilisable pour former l'enveloppe du conteneur, on peut citer les matériaux tissés ou les matériaux non-tissés. La matière utilisable pour former l'enveloppe des conteneurs peut être d'origine naturelle telle que minérale, végétale ou animale, ou encore d'origine synthétique. Comme matière on peut citer, à titre d'exemple non limitatif, le polypropylène, les polyesters, les polyamides, l'aluminium, le cuivre, le titane, le nickel, le platine, l'acier inoxydable ou un grillage ou treillis métallique, les dimensions des ouvertures ou maille de ce grillage ou treillis étant telles que définies ci-avant.

La matière choisie sera de préférence physiquement et chimiquement inerte vis-à-vis des fluides et des solides avec lesquels elle entre en contact.

Lorsque la matière choisie pour former l'enveloppe du conteneur n'a pas de caractéristiques mécaniques suffisantes pour permettre d'obtenir un conteneur capable de résister au poids de l'ensemble des conteneurs de la zone d'alkylation, on inclura des éléments de renforcement mécanique tels que par exemple des tiges d'acier ou tout autre moyen connu des hommes du métier permettant de fabriquer un conteneur ayant les caractéristiques mécaniques souhaités.

Dans une des formes préférée de réalisation de l'invention les conteneurs auront des caractéristiques mécaniques suffisantes pour que les conteneurs disposés en bas de réacteur ne soient pas écrasés, sous l'action du poids des conteneurs supérieurs. De cette manière les mouvements de solide à l'intérieur du conteneur peuvent se produire dans l'ensemble des conteneurs situés sur toute la hauteur de réaction.

La zone d'alkylation peut comprendre un ou plusieurs réacteurs contenant chacun un ou plusieurs lits fixes de conteneurs ou de chaînes de conteneurs. Cette zone comprendra avantageusement pour chaque réacteur ou pour l'ensemble des réacteurs des moyens de refroidissement, d'au moins l'un des réactifs sur la ligne d'introduction dudit réactif dans ladite zone ou dans ledit réacteur. Les réacteurs pourront être disposés en série ou en parallèle selon les règles de l'art.

Selon un mode de réalisation particulier chaque réacteur comprendra une pluralité de points d'introduction de la charge comprenant au moins une oléfine.

Les figures jointes illustrent l'invention, et plus précisément une des mises en oeuvre préférée du procédé selon l'invention, sans en limiter la portée.

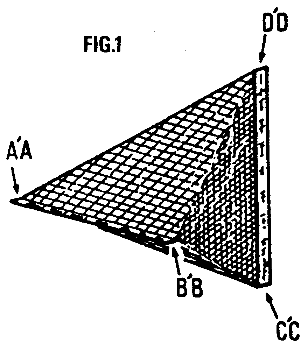

La figure 1 illustre en perspective un conteneur de forme tétraédrique ABCD dont les quatre faces sont réalisées en matériau poreux, par exemple un tissu métallique. On remarque entre A et B et entre C et D l'existence d'un bourrelet AA'BB' résultant de la soudure des deux bases communes des triangles ABC et ABD d'une part et ACD et BCD d'autre part.

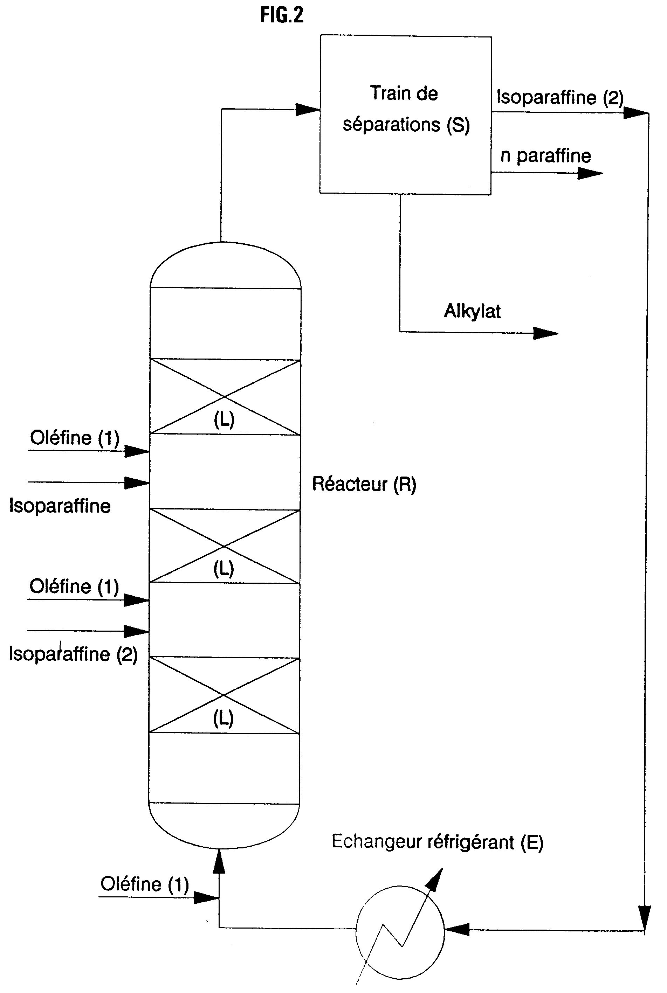

La figure 2 illustre la mise en oeuvre de l'invention dans un réacteur (R) comportant plusieurs lits (L) (quatre lits) de conteneurs renfermant le catalyseur d'alkylation. La circulation des fluides dans le réacteur (R) se fait dans le cas illustré ici de bas en haut. L'injection de la charge oléfinique par des lignes (1) et de l'isoparaffine par des lignes (2) est étagée tout au long du réacteur. Cette injection est effectuée entre deux lits (L) consécutif de conteneur contenant le catalyseur. Le réacteur comporte un échangeur réfrigérant (E) sur la ligne 2 de recyclage de l'isoparaffine vers la partie basse du réacteur. L'effluent sortant du réacteur par son sommet est envoyé dans un train de séparation (S) à partir duquel on récupère l'alkylat recherché, de la n-paraffine et de l'isoparaffine par une ligne 2 que l'on recycle en bas du réacteur.

### EXEMPLE

Un test d'alkylation est effectué dans une installation comme celle schématisé sur la figure 2 dans les conditions suivantes qui permettent de produire 10 kilo par heure (kg/h) d'alkylat. L'oléfine utilisée est contenue dans un mélange d'hydrocarbures dont la composition pondérale est la suivante :
28 % de butène 1,
34 % de butène 2,
0,5 % d'isobutène
22 % d'isobutane
14 %de n-butane et
1,5 % de composés ayant 3 ou 5 atomes de carbone dans leur molécule.

L'isoparaffine est de l'isobutane sensiblement pur. Ces réactifs sont préalablement à leur introduction dans le réacteur séchés et désoxygénés. Le catalyseur employé est de la silice imprégnée par de l'acide sulfurique à 96 % en poids. La taille moyenne des particules solides de catalyseur est de 70x10⁻⁶ m. Chaque conteneur tétraédrique régulier a un coté de 1 cm et est formé à partir d'un grillage en acier inox dont les mailles ont une ouverture de 30x10⁻⁶ m. Chaque conteneur contient 0,1 centimètres cube de catalyseur. Le volume total réactionnel est de 42 litres. Le réacteur a un diamètre de 0,15 m. Il comporte trois lits de conteneurs et trois points d'injection d'une charge formée par le mélange d'hydrocarbure contenant du butène décrit ci-devant dilué par de l'isobutane. Dans chaque lit les conteneurs sont disposés en vrac. La proportion en poids de l'isobutane par rapport au poids des butènes contenu dans le mélange introduit dans le réacteur est de 333 : 1. Le débit total de butènes introduit dans le réacteur est de 5 kg/h et celui d'isobutane de 1600kg/h.

La température opératoire varie entre le bas et le haut du réacteur de -7 °C à - 5 °C. La perte de charge mesurée est de 0, 03 mégapascal.

On obtient un alkylat dont la composition pondérale est la suivante :

| | |
|---|---|
| Paraffine ayant de 5 à 7 atomes de carbone : | 5,5 % |
| Paraffine à 8 atomes de carbone | 92 % |
| Paraffine ayant plus de 9 atomes de carbone | 2,5 % |

La teneur pondérale en triméthylpentanes dans la fraction C8 est de 91,5 %. La conversion des butènes est de 99,8 %.

## Revendications

**1 -** Procédé d'alkylation d'au moins une oléfine par au moins une isoparaffine en présence d'un catalyseur d'alkylation sous forme de particules solides caractérisé en ce que la réaction est effectuée dans une zone d'alkylation contenant une pluralité de conteneurs renfermant chacun une quantité de particules solides catalytiques telle que le volume Va de ces particules mesuré après leur mise en contact avec les réactifs dans les conditions de la réaction d'alkylation soit supérieur à zéro et inférieur ou égal au volume interne Vt dudit conteneur, lesdits conteneurs ayant chacun une enveloppe externe perméable et résistante aux fluides dans les conditions de la réaction et imperméable auxdites particules solides catalytiques et ayant des caractéristiques mécaniques suffisantes pour résister, sans déformation excessive, à la charge de l'ensemble des conteneurs présents dans ladite zone d'alkylation.

**2 -** Procédé selon la revendication 1 dans lequel la zone d'alkylation contient une pluralité de conteneurs renfermant chacun une quantité de particules solides catalytiques telle que le volume Va de ces particules mesuré après leur mise en contact avec les réactifs dans les conditions de la réaction d'alkylation soit supérieur à zéro et strictement inférieur au volume interne Vt dudit conteneur les renfermant.

**3 -** Procédé selon la revendication 1 ou 2 dans lequel le catalyseur est choisi dans le groupe formé par : un catalyseur comprenant un support solide organique ou minéral imprégné par au moins un acide minéral et par un catalyseur comprenant au moins un halogénure choisi dans le groupe formé par les halogénures de bore et les halogénures d'aluminium ledit halogénure étant associé à au moins un composé choisi dans le groupe formé par les halogénures d'ammonium quaternaire et les halohydrates d'amine.

**4 -** Procédé selon l'une des revendications 1 à 3 dans lequel le catalyseur comprend un support organique ou minéral, et de préférence minéral, imprégné par de l'acide sulfurique.

**5 -** Procédé selon l'une des revendications 1 à 4 dans lequel le catalyseur est sous forme de particules solides granulaires ayant un diamètre moyen d'environ 20x10⁻⁶ à environ 400x10⁻⁶ mètre.

**6** Procédé selon l'une des revendications 1 à 5 dans lequel chaque conteneur comporte quatre faces sensiblement triangulaires constituant sensiblement un tétraèdre et dans lequel lesdits conteneurs sont disposés en vrac dans la zone d'alkylation.

**7 -** Procédé selon l'une des revendications 1 à 5 dans lequel chaque conteneur est relié à au moins un conteneur contigu, de manière à former une chaîne de conteneur et en ce que la zone d'alkylation comprend au moins une chaîne de conteneurs.

**8 -** Procédé selon l'une des revendications 1 à 7 dans laquelle la zone d'alkylation comprend un ou plusieurs réacteurs contenant chacun un ou plusieurs lits fixes de conteneurs ou de chaînes de conteneurs.

**9 -** Procédé selon la revendication 8 dans lequel chaque réacteur comporte des moyens de refroidissement, d'au moins l'un des réactifs sur la ligne d'introduction dudit réactif dans ledit réacteur.

**10 -** Procédé selon la revendication 8 ou 9 dans lequel chaque réacteur comprend une pluralité de points d'introduction de la charge comprenant au moins une oléfine.
